Europäisches Patentamt

(19) **European Patent Office**

**Office européen des brevets**

(11) Numéro de publication : **0 056 202**
**B1**

(12) **FASCICULE DE BREVET EUROPÉEN**

(45) Date de publication du fascicule du brevet :
21.10.87

(51) Int. Cl.⁴ : **C 12 M 1/00**, C 02 F 3/28

(21) Numéro de dépôt : **81401672.1**

(22) Date de dépôt : **23.10.81**

(54) **Digesteur horizontal pour la production continue de gaz méthane.**

(30) Priorité : **12.12.80 FR 8026985**

(43) Date de publication de la demande :
**21.07.82 Bulletin 82/29**

(45) Mention de la délivrance du brevet :
**21.10.87 Bulletin 87/43**

(84) Etats contractants désignés :
**BE CH DE GB IT LI NL**

(56) Documents cités :
**DE-C- 884 176**
**FR-A- 896 517**
**FR-A- 914 808**
**FR-A- 987 924**
**FR-A- 1 009 247**
**FR-A- 1 367 006**
**FR-A- 2 305 113**
**FR-A- 2 453 588**

(73) Titulaire : **Nayet, Roger**
**Les Boissonnats**
**F-89350 Champignelles (FR)**

**Nayet née Roy, Françoise**
**Les Boissonnats**
**F-89350 Champignelles (FR)**

(72) Inventeur : **Nayet, Roger**
**Les Boissonnats**
**F-89350 Champignelles (FR)**
Inventeur : **Nayet née Roy, Françoise**
**Les Boissonnats**
**F-89350 Champignelles (FR)**

Jouve, 18, rue St-Denis, 75001 Paris, France

## Description

La présente invention concerne le principe d'un digesteur horizontal mixte, en vue de la production continue de gaz méthane.

Dans les appareils connus à ce jour travaillant en continu, seules les matières liquides ou broyées peuvent être traitées. Pour éviter l'inconvénient de formation de croûte ceux-ci ont recours à des systèmes d'agitation complexes. Le traitement des matières solides n'étant effectué qu'en discontinu avec fermentation aérobie séparée nécessitant une manipulation manuelle supplémentaire.

Dans FR-A-2 453 588 est décrit un digesteur de forme tunnel dans lequel le gaz est récupéré dans la partie haute du tunnel formant hotte. La section du tunnel est variable et fonction du débit choisi. Le document FR-A-2 305 113 décrit l'utilisation des gaz chauds aspirés pour réchauffer le grand compartiment du digesteur. L'apport d'air se fait par aspiration.

Le digesteur selon l'invention permet, en introduisant les matières à traiter à une extrémité, de leur faire subir une fermentation aérobie, avec possibilité de récupération de chaleur. Puis entraînées par un mouvement continu celles-ci pénètrent dans le liquide pour y commencer une fermentation anaérobie avec production de gaz. Par ce même mouvement elles sont acheminées vers l'autre extrémité où elles sortent digérées. Le tout sans aucune manipulation manuelle. Les matières digérées peuvent être utilisées comme fertilisant pour les cultures.

Ce digesteur peut être conçu :
soit de forme droite, les matières entrant à une extrémité (A) et sortant à l'autre extrémité (C),
soit de forme U, les sections (A) et (C) se trouvant côte à côte,
soit de forme ronde ou ovale (fig. H), les sections (A) et (C) se trouvant face à face, permettant un mouvement continu du système d'avancement. Toutes les formes peuvent convenir à la section de ce digesteur. Cette forme laisse au centre de l'ensemble du digesteur un réservoir (25) pouvant servir de fosse de décantation et d'épuration aux liquides traités.

Des moulinets entraînés par le passage des « râteaux » ou un rehaussement articulé de la partie supérieure de ceux-ci, brassent la couche supérieure et évite ainsi la formation d'une croûte.

Le digesteur est fermé par un couvercle en un ou plusieurs éléments. Les bords de ceux-ci descendent suffisamment dans le liquide afin de former un joint hydraulique.

La conception de l'appareil permet de traiter des matières soit liquides (lisiers, boues) soit semi-liquides (lisiers, boues enrichies de matières organiques) soit solides (fumier, paille) même non broyées.

Les dimensions et volume de l'appareil sont calculés en fonction des matières à digérer, tant en quantité que qualité.

Tel qu'il est présenté le digesteur comprend trois sections

Section A : fermentation aérobie

La capacité de cette section est calculée en fonction :
du temps de passage (2 à 10 jours) selon la nature des matières,
de l'évolution de celles-ci à l'intérieur du digesteur,
de la fermentation qui peut être accélérée par le mouvement ascendant de l'air chaud qui pénètre en partie basse à l'aide des passages (12) réservés à cet usage.

L'inclinaison de cette section (A1) peut varier de 40 à 90° (14) selon l'emplacement et la nature des matières à digérer. Cette section peut se concevoir horizontale au départ (29), puis en pente accélérée (30) pour pénétrer dans le liquide puis en section (B).

La récupération d'une partie de la chaleur de cette fermentation se fait par le serpentin (13) fixé dans le pourtour. Par l'intermédiaire des canalisations (9) dans le fond du digesteur ces calories peuvent servir, en complément d'un réchauffeur classique, à maintenir ou augmenter la température de la masse dans la partie principale du digesteur.

Une humidification des matières en fermentation aérobie peut se faire pendant le chargement ainsi que lors des temps intermédiaires.

L'entrée des matières à digérer (lisiers, boues etc) se fait par l'ouverture (10) qui se trouve en partie basse de la section (A), au-dessus du niveau (8) du liquide du digesteur.

Dans le cas d'utilisation de matières liquides, la section aérobie peut être partiellement ou entièrement supprimée.

Section B : fermentation anaérobie

Les dimensions et la capacité sont calculées en fonction de la nature des matières à traiter, de leur volume, de leur temps de passage variable selon la température intérieure du digesteur et les besoins en énergie.

Une isolation (14) aussi parfaite que possible sera réalisée dans les parois et le fond (15) de l'appareil qui peuvent être en béton, plastique ou autre matériau.

La fermentation anaérobie commence dès que les matières à digérer pénètrent dans le liquide du digesteur. Leur avancement à l'intérieur est assuré soit par un système de « râteaux » soit, par un système de « containers ».

Les râteaux (3) de dimensions légèrement inférieures à la section du digesteur, sont composés de dents rapprochées (fig. D3) de manière à entraîner les boues se déposant dans le fond, la partie basse des râteaux est légèrement courbée vers l'avant (B3).

Ces râteaux seront guidés soit par dispositif

2

dans les parois latérales (16 et 17), soit par glissière dans le fond (22). Ce système permet d'épouser les différentes formes d'entrée et sortie du digesteur.

Le mouvement de ces râteaux peut être soit continu, alterné ou en va-et-vient. Dans ce dernier cas les râteaux seront articulés de manière à s'éclipser en mouvement retour, et reprendre la position initiale en mouvement aller.

Au passage ces râteaux entraînent des moulinets (fig. B4) qui par leur mouvement brassent la couche supérieure et empêchent la formation d'une croûte.

Si les moulinets sont efficaces pour briser la croûte formée au niveau du liquide pour des matières courtes (paille broyée, lisier, menu branchage, etc) ; dans le cas de traitement de matières ligneuses, longues, un enroulement peut se produire autour des moulinets. Pour palier à cet inconvénient, un rehaussement de la partie supérieure des râteaux (fig. E18) remplace les moulinets. Cette partie (18) est alors articulée pour permettre un basculement vers l'avant entraînant, par ce mouvement, toutes les matières se trouvant en surface. Ce mouvement permet le passage des obstacles formés par les bords des couvercles ou butées (27) et brise la croûte de façon efficace.

Ce mouvement est commandé par une glissière (fig. E19) fixée dans la paroi du digesteur et positionnée de façon que la commande du mouvement s'effectue au moment opportun pour le passage des butées. Un galet (fig. E20) placé à la partie basse de l'articulation et à l'extérieur du râteau vient prendre appui sur cette glissière. Ainsi guidé, celui-ci oblige la partie haute du râteau à basculer vers l'avant comprimant, vers le bas, toutes les matières à ce niveau.

Les butées (27) forment des parois transversales dans la partie supérieure du digesteur, plongent dans le liquide et servent :

d'une part à maintenir les matières remontant en surface de façon à éviter leur fuite en avant lors du mouvement de l'articulation du râteau

d'autre part de joints hydrauliques.

Ces mêmes râteaux peuvent être accouplés deux par deux (fig. F) par des tubes ou autres matériaux, de chaque côté et au fond, formant cloisons à claires-voies (fig. F23) et constituant ainsi des containers, qui, reliés les uns aux autres forment un ensemble pour entraîner les matières.

Le fond de ces containers peut être à claires-voies larges si l'on désire un déchargement des matières digérées par gravité à la sortie de la section (C), ou à claires-voies étroites dans le cas du transport des matières digérées dans le container. Dans ce cas la possibilité d'un fond ouvrant pourra être retenue. Cette réalisation apporte, dans certains cas, un avantage pour la manipulation des matières et serait une solution pour éviter le retour des râteaux dans le cas d'un digesteur droit à mouvement continu.

Ces containers assurent le même travail que les râteaux à l'intérieur du digesteur. Seul l'arrière du container sera équipé d'un rehaussement articulé. Ils seront guidés de façon identique aux râteaux.

Leurs dimensions sont variables et appropriées à chaque type de digesteur.

L'entrée (A) et la sortie (C) du digesteur auront des angles effacés (26) pour éviter le freinage de l'avancement des matières.

Le mouvement des différents systèmes d'avancement des matières à l'intérieur du digesteur peut être commandé par la force motrice d'une turbine alimentée par la pression de sortie du gaz du digesteur. Dans ce cas la hauteur du joint hydraulique des couvercles devra être suffisante : minimum 40 cm.

La partie supérieure de la section (B) est fermée par des couvercles (5) en un ou plusieurs éléments dont les bords, plongeant suffisamment dans le liquide, assurent un joint hydraulique efficace. Sur la partie supérieure de chaque couvercle est fixée la sortie (6) des gaz. Ces différentes sorties sont reliées par une canalisation acheminant le gaz vers la turbine puis l'utilisation. Sur chaque couvercle il est prudent d'y adjoindre une soupape de sécurité (7).

Le fait de concevoir un couvercle en plusieurs éléments donne la possibilité de contrôler la production de gaz suivant l'évolution des matières en cours de digestion, d'où la possibilité d'accélérer ou ralentir le mouvement selon la production constatée. Cette disposition permet également une intervention localisée sans perturbation des autres sections.

Le digesteur peut également être construit d'un seul corps, les parties supérieures et inférieures étant constituées d'un seul ensemble sans raccord (E21). Cette conception évite l'usage d'un couvercle et convient aux différentes formes et sections de digesteurs. Si ce genre de digesteur évite les joints hydrauliques des couvercles, il nécessite quand même à chaque extrémité une retombée (fig. G24) plongeant suffisamment dans le liquide afin de former un joint hydraulique pour la retenue du gaz à l'intérieur. Le dessus de ce digesteur est libre de forme, ne subissant aucune contrainte due au système d'avancement. Ce type de construction peut convenir aux différents systèmes d'avancement des matières à l'intérieur du digesteur.

Section C : sortie des matières digérées

Les matières entraînées par les râteaux ou les containers sortent du digesteur par la section (C) dont la pente peut être prolongée et épouser différentes courbes (29 et 30).

Ces matières digérées pourront être utilisées comme fertilisant.

Les liquides sont évacués par l'orifice (11) muni d'une grille. Celui-ci est placé à une hauteur bien définie pour maintenir constant le niveau (8) du liquide à l'intérieur du digesteur, afin d'assurer l'efficacité des joints hydrauliques, et pour évacuer les liquides d'égouttage.

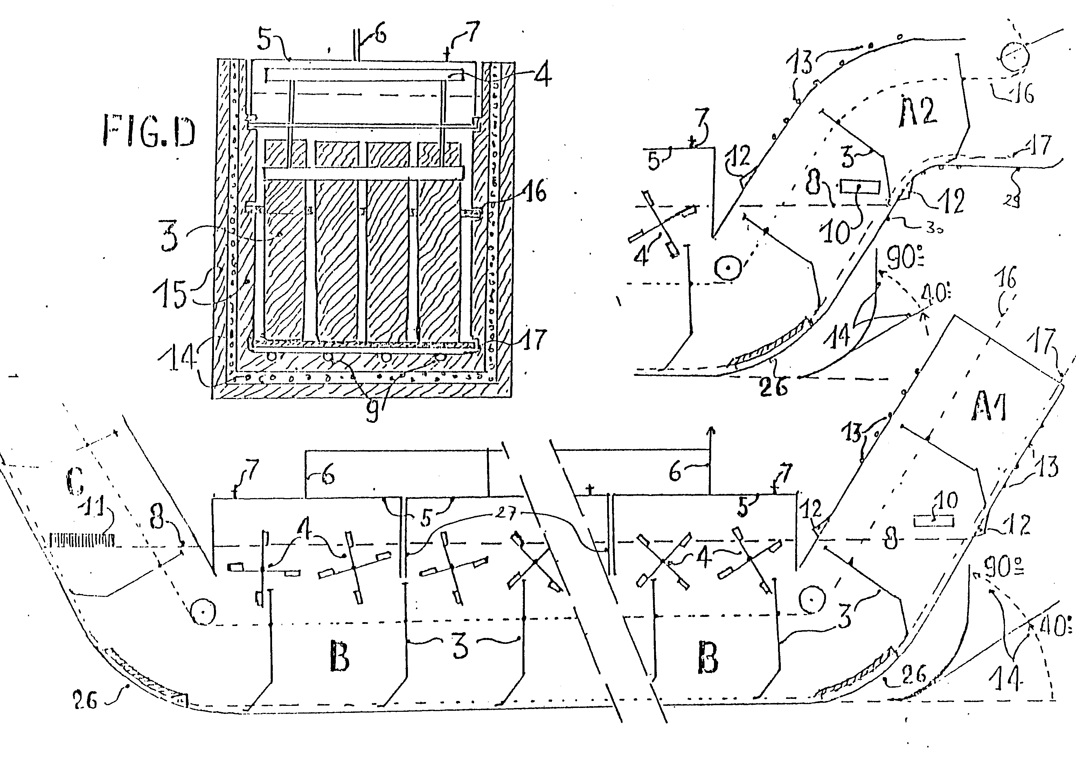

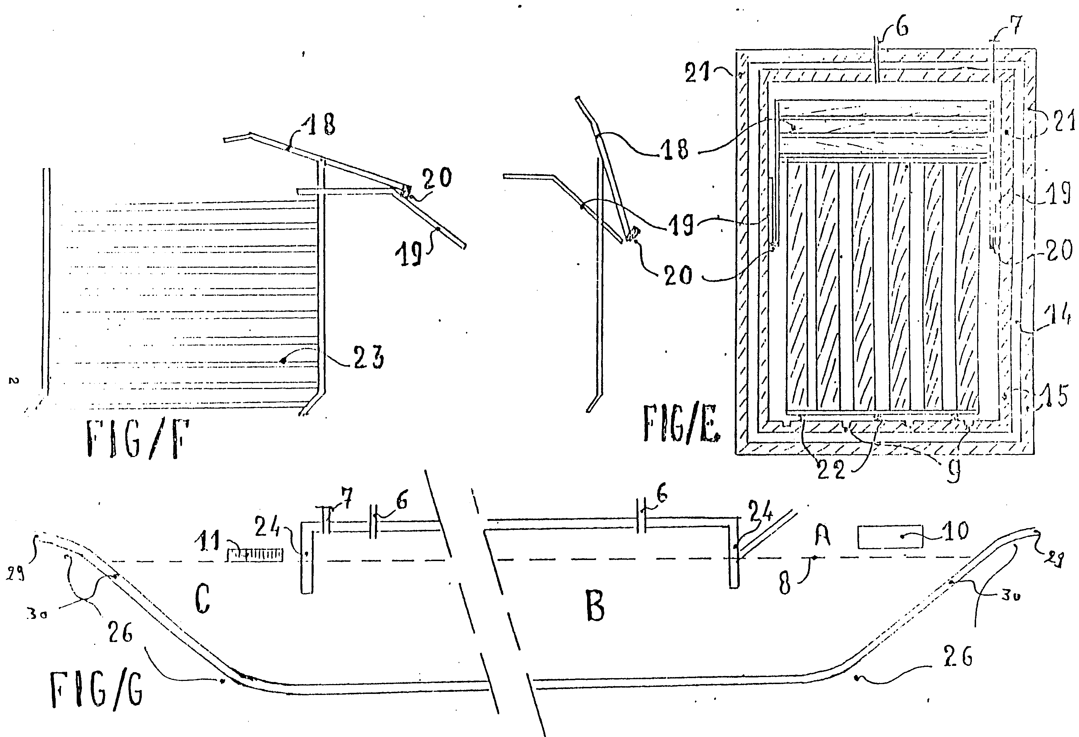

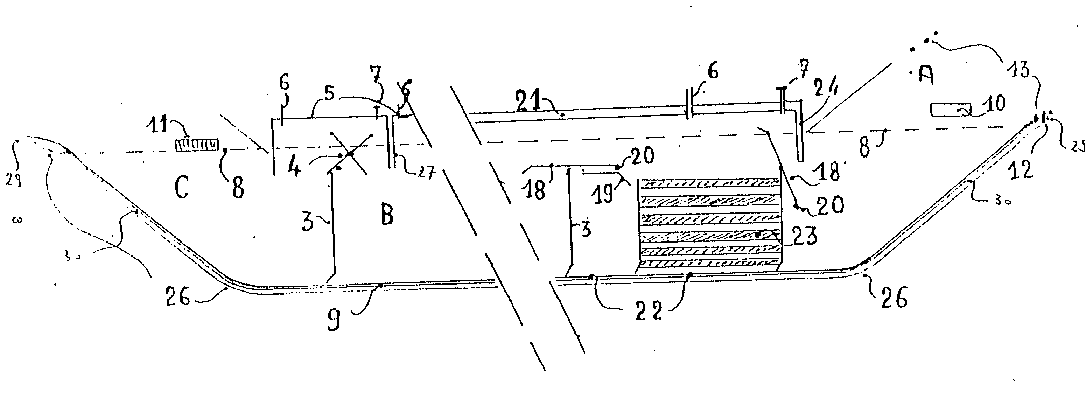

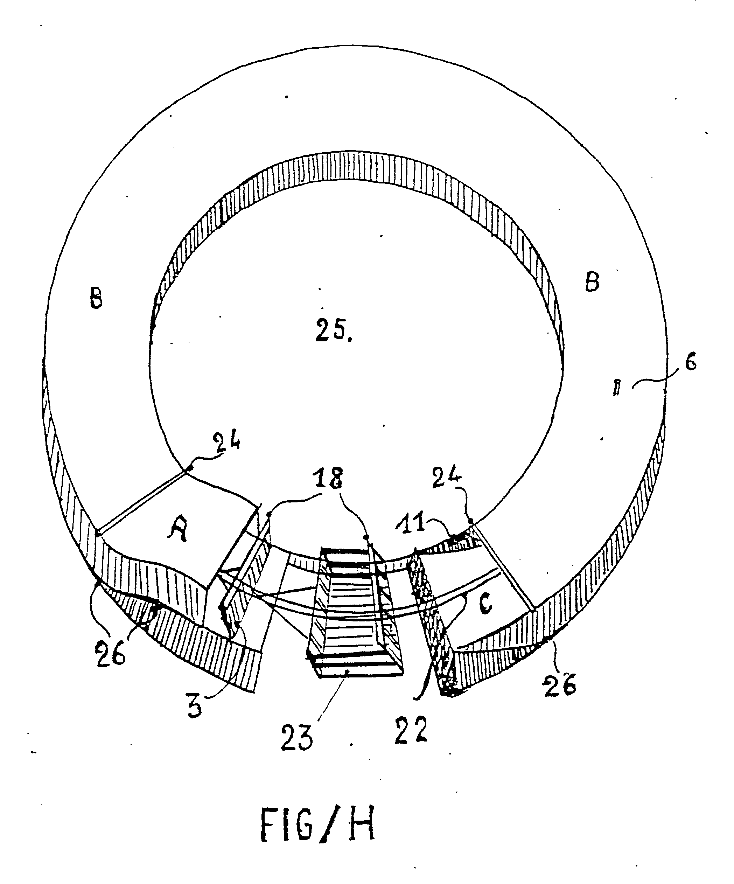

**Revendications**

1. Digesteur horizontal mixte en vue de la production continue de gaz méthane, caractérisé par le fait que les matières de différentes natures comprenant des matières ligneuses, longues des matières courtes, des boues enrichies de matières organiques et des lisiers sont introduites à la section (A) du digesteur. Un dispositif d'entraînement constitué de râteaux (3) ou de containers (23) guidés soit dans les parois latérales (16 et 17) soit par le fond (22) épouse les différentes formes de digesteur couloir droit (D) à circulaire (H). La partie inférieure des râteaux courbée vers l'avant (3) entraîne les matières lourdes se déposant dans le fond du digesteur. La croûte se formant en surface est brisée soit par les moulinets (4), soit par le rehaussement articulé (18) des râteaux. Les bords des couvercles ou butées (27) empêchent les matières flottant en surface de s'éclipser vers l'avant. La sortie des liquides traités et le maintien du niveau constant, assurant l'efficacité des joints hydrauliques, sont tributaires d'un orifice (11) placé à un endroit précis qui évacue également les liquides d'égouttage.

2. Digesteur selon revendication 1, caractérisé par le fait qu'un serpentin (13) récupère la chaleur dégagée en fermentation aérobie et la restitue en section (B) par des canalisations (9) placées au fond de celle-ci.

3. Digesteur selon revendication 1, caractérisé par le fait que le système d'entraînement constitué de râteaux (3) ou de containers (23) est guidé par glissières.

4. Digesteur selon revendications précédentes, caractérisé par le fait que le système d'entraînement permet de positionner sous des angles différents :
la section (A) par rapport à la section (B),
la section (C) par rapport à la section (B).

5. Digesteur selon revendications précédentes, caractérisé par le fait que le système d'entraînement est composé de râteaux (3) à claires-voies qui entraînent les matières de la partie aérobie à la sortie. Ces râteaux peuvent être accouplés deux par deux et former des containers (F23).

6. Digesteur selon revendications précédentes, caractérisé par le fait que les râteaux ou les containers actionnent au passage, des moulinets (4) brisant la croûte se formant à la surface du liquide et assurant un brassage de la masse.

7. Digesteur selon revendications précédentes, caractérisé par le fait que les râteaux ou les containers sont munis d'un rehaussement articulé (18) en partie supérieure qui se rabat en avant pour le passage des butées (27), et, dans le même mouvement comprime vers le bas les matières se trouvant à ce niveau brisant ainsi la croûte.

8. Digesteur selon revendications précédentes, caractérisé par le fait que les matières remontant en surface sont maintenues par les bords des couvercles ou butées (27). Ces butées servent également de joints hydrauliques dans l'option d'un couvercle en plusieurs éléments.

**Claims**

1. Mixed horizontal digester for the continuous production of methane gas, distinguished by the fact that the different types of material — comprising long ligneous material, short ligneous material, sludge enriched with organic matter and liquid manure — are inserted in section (A) of the digester ; a drive device consisting of forks (3) or containers (23) guided along the side panels (16 and 17) or along the base (22) are moulded to the different digester shapes, from straight chute (D) to circular chute' (H). The lower part of the forks curved foward (3) carries along the heavy matter deposited at the bottom of the digester ; the crust forming on the surface is broken up either by paddles (4) or by the hinged raising (18) of the forks ; the edges of the covers or abutments (27) prevent surface-floating matter from being carried forward ; the discharge of treated liquids and the maintenance at a constant level, ensuring the efficiency of the hydraulic seals, are dependent on an orifice (11) placed in a precise position and through which the drainage liquids are also discharged.

2. Digester as per claim 1, distinguished by the fact that a pipe coil (13) recovers the heat given off during aerobic fermentation and restores it in section (B) through ducting (9) at the bottom of the latter.

3. Digester as per claim 1, distinguished by the fact that the drive system, consisting of forks (3) or containers (23), is guided by slides.

4. Digester as per preceding claims, distinguished by the fact that the drive system can be used to position at different angles :
section (A) in relation to section (B),
section (C) in relation to section (B).

5. Digester as per preceding claims, distinguished by the fact that the drive system comprises slatted forks (3) which carry the material along from the aerobic part to the discharge point. These forks can be coupled two by two and form containers (23).

6. Digester as per preceding claims, distinguished by the fact that, during their movement, the forks or containers actuate the paddles (4), which break up the crust forming on the surface of the liquid and stir the whole volume.

7. Digester as per preceding claims, distinguished by the fact that the forks or containers are fitted with a hinged raising part (18) at the top which folds forward to pass the abutments (27) and, with the same movement, pushes down the materials at that point, thus breaking the crust.

8. Digester as per preceding claims, distinguished by the fact that materials rising to the surface are held by the edges of the covers or abutments (27). These abutments also serve as hydraulic seals in the version with a cover in several sections.

**Patentansprüche**

1. Horizontale Digeriervorrichtung, gemischt, für die fortlaufende Erzeugung von Methangas, dadurch gekennzeichnet, dass die verschiedenartigen Materien, die lange holzige Materien, kurze Materien, mit organischen Materien angereicherte Schlämme und Güllen enthalten, in den Abschnitt (A) der Digeriervorrichtung eingeführt werden ; eine Antriebsvorrichtung, bestehend aus Rechen (3) oder Containern (23), entweder in Seitenwänden (16 und 17) oder am Boden (22) geführt, passt sich den verschiedenen Formen der Digeriervorrichtung an, mit geradem (D) bis kreisförmigem (H) Gang. Der untere Teil der nach vorn gekrümmten Rechen (3) führt die schweren Stoffe mit, die sich am Boden der Digeriervorrichtung ablagern ; die sich an der Oberfläche bildende Kruste wird durch Räder (4) oder durch gegliederte Anhebung (18) der Rechen gebrochen ; die Ränder der Deckel oder Anschläge (27) verhindern die an der Oberfläche schwimmenden Materien, nach vorn auszutreten ; der Ausgang der behandelten Flüssigkeiten und das konstant gehaltene Niveau, das die Wirksamkeit der hydraulischen Dichtungen gewährleistet, werden durch eine Öffnung (11) bewirkt, die an einem präzisen Punkt angeordnet ist, an dem auch die Abtropfflüssigkeiten ausgeschieden werden.

2. Digeriervorrichtung gemäss Anspruch 1, dadurch gekennzeichnet, dass eine Schlange (13), die durch die Aerobiegärung freiwerdende Wärme aufnimmt und im Querschnitt (B) durch Leitungen (9) auf deren Boden abgibt.

3. Digeriervorrichtung gemäss Anspruch 1, dadurch gekennzeichnet, dass das Antriebssystem, bestehend aus Rechen (3) oder Containern (23), durch Führungsbahnen geführt wird.

4. Digeriervorrichtung nach den vorangehenden Ansprüchen, dadurch gekennzeichnet, dass das Antriebssystem gestattet, unter verschiedenen Winkeln zu positionieren

den Schnitt (A) in Bezug auf den Schnitt (B),
den Schnitt (C) in Bezug auf den Schnitt (B).

5. Digeriervorrichtung nach den vorangehenden Ansprüchen, dadurch gekennzeichnet, dass das Antriebssystem aus Rechen (3) mit Lücken besteht, welche die Stoffe des Aerobieteils zum Ausgang befördern. Diese Rechen können paarweise gekoppelt werden und Container bilden (F23).

6. Digeriervorrichtung nach den vorangehenden Ansprüchen, dadurch gekennzeichnet, dass die Rechen- oder die Container bei ihrem Durchgang Räder (4) bewegen, welche die sich an der Oberfläche der Flüssigkeit bildende Kruste brechen und eine Vermischung der Masse gewährleisten.

7. Digeriervorrichtung nach den vorangehenden Ansprüchen, dadurch gekennzeichnet, dass die Rechen oder Container mit einer gegliederten Erhöhung (18) am oberen Teil versehen sind, die sich für den Durchgang der Anschläge (27) nach vorne klappen lässt, und die in der gleichen Bewegung die Stoffe in dieser Ebene nach unten drückt und dadurch die Kruste bricht.

8. Digeriervorrichtung nach den vorangehenden Ansprüchen, dadurch gekennzeichnet, dass die an die Oberfläche aufsteigenden Materien durch die Ränder der Deckel oder Anschläge (27) festgehalten werden. Diese Anschläge dienen in der Option eines mehrteiligen Deckels auch als hydraulische Dichtungen.

FIG.D

FIG/F

FIG/E

FIG/G

0 056 202

0 056 202

FIG/H